# EUROPEAN PATENT APPLICATION

(11) **EP 2 894 472 A1**
(43) Date of publication of application: **15.07.2015**
(21) Application number: 12884337.2
(22) Date of filing: 05.09.2012
(51) Int. Cl.: G01N 33/50, G01N 33/15

(54) **METHOD FOR SCREENING MATTER ACTING ON EPITHELIAL MAINTENANCE OF CELLS**

(71) Applicant: JSR Corporation, Minato-ku Tokyo 105-8640 (JP)
(72) Inventor: ITOH, Manabu, Kawasaki-shi Kanagawa 213-0012 (JP); ARAI, Kazuya, Kawasaki-shi Kanagawa 213-0012 (JP); KAJITA, Hiroshi, Kawasaki-shi Kanagawa 213-0012 (JP); TANAKA, Satoru, Kawasaki-shi Kanagawa 213-0012 (JP)
(74) Representative: Gille Hrabal
(86) International application number: PCT/JP2012/072657
(87) International publication number: WO 2014/038025

(57) **Abstract**

A screening method for a substance acting on a maintenance of the epithelial properties of cells which enables obtainment of highly-reliable data quickly at low costs is provided. The screening method includes (a) a step for culturing cells on a cell culture substrate that can form a spheroid, (b) a step for causing the cells to contact a test substance, and (c) a step for evaluating an effect of the test substance acting on a maintenance of the epithelial properties of the cells with a change in a morphology of the spheroid being as an indicator. In this case, the step (c) can carry out an evaluation through a measurement of the number of spheroids in a predetermined size or a measurement of the hypoxic areas in the spheroid.

## Description

### Technical Field

The present invention relates to a method of screening a substance that acts on a maintenance of the epithelial properties of cells with a change in a morphology of spheroid being as an indicator.

### Background Art

Epithelial-Mesenchymal Transition (EMT) is a phenomenon in which a cell cannot maintain properties as an epithelium, and obtains properties as a mesenchymal.

In recent years, it is reported that epithelial-mesenchymal transition relates to invasion and metastasis of a cancer cell, and remodeling and fibrosis of a tissue, etc. Hence, it is expected that substances which acts on a maintenance of the epithelial properties of cells, i.e., substances which inhibits the epithelial-mesenchymal transition, and substances inducing Mesenchymal-Epithelial Transition (MET) that is a reverse phenomenon of the epithelial-mesenchymal transition result in a remedy of cancer and fibrosis, etc.

Conventionally, as a technique of screening substances that act on a maintenance of the epithelial properties of cells, a method is known which causes a test substance to contact a cell to which epithelial-mesenchymal transition is induced, and which evaluates a change in a biomarker level indicating an epithelial-mesenchymal transition condition as an indicator (see, for example, Patent Literature 1).

### Prior Art Literature

### Patent Literature

Patent Literature 1: International Publication No. WO 2009/111067

### Disclosure of Invention

### Problem to be Solved by the Invention

A screening method using a biomarker level as an indicator needs, however, a large amount of preparation and costs for detection. Accordingly, it is difficult to apply such a method to a screening that needs a speedy evaluation for a plurality of samples like a screening in the early stage of a drug development. Moreover, it is difficult to detect a biomarker of a low concentration, and thus the reliability is poor.

Hence, it is an object of the present invention to provide a screening method which can be carried out quickly and at a low cost, and which can obtain highly-reliable data for a substance that acts on a maintenance of epithelial properties of cells

### Means for Solving the Problem

The inventors of the present invention found a correlation between the expression level of E-cadherin that is an cell adhesion factor expressed in an epithelial cell and a behavior indicated by a cell cultured on a predetermined cell culture substrate. That is, when the expression level of E-cadherin decreases due to the epithelial-mesenchymal transition and a cell becomes unable to maintain the epithelial properties, such a cell obtains a high migration capability, and becomes a condition to be likely to form a spheroid on the predetermined cell culture substrate, and the formed spheroid indicates a different morphology in accordance with the expression level of E-cadherin. The inventors of the present invention have accomplished the present invention that is a screening method with a change in the morphology of a spheroid being as an indicator.

The subject matters of the present invention are follows.

A screening method for a substance acting on a maintenance of an epithelial properties of cells according to the present invention includes: (a) a step for culturing cells on a cell culture substrate that can form a spheroid; (b) a step for causing the cells to contact a test substance; and (c) a step for evaluating an effect of the test substance acting on a maintenance of the epithelial properties of the cells with a change in the morphology of the spheroid being as an indicator.

In this case, the step (c) may carry out an evaluation through a measurement of a number of spheroids in a predetermined size.

In this case, also, the step (c) may carry out an evaluation using a reagent that can detect a hypoxic area in a spheroid.

The step (a) may be carried out on a cell culture substrate comprising a predetermined concavo-convex structure that functions as a cell adhesion surface.

In this case, the concavo-convex structure may include a plurality of unit structures disposed with regularity, the unit structure being formed in a predetermined planar shape.

The concavo-convex structure may include a plurality of unit structures disposed with regularity, a width between the unit structures being equal to or smaller than 3 µm, the unit structure being formed in a polygonal shape in a plane direction, and a minimum internal diameter of the unit structure being equal to or smaller than 3 µm.

### Effect of the Invention

The screening method of the present invention can perform screening of a target substance quickly and at a low cost, since such a method performs evaluation with a change in a morphology of a spheroid as an indicator. Moreover, the method utilizes a spheroid that is expected to reflect a condition further similar to condition in a biological object than mono layer cells, thereby enabling screening efficiently. Furthermore, since it is possible to perform evaluation while capturing a plurality of indicators simultaneously, the method can obtain a highly-reliable screening result.

### Brief Description of Drawings

FIG. 1 is a plan view illustrating a concavo-convex structure of a cell culture substrate to be used in a method of the present invention;
FIG. 2 is a photograph illustrating morphologies of spheroids;
FIG. 3 is a photograph and a graph illustrating E-cadherin expression levels;
FIG. 4 is a microscope photograph illustrating an effect of an epithelial-mesenchymal transition inhibitor acting on morphologies of spheroids;
FIG. 5 is a graph illustrating signal intensities of a hypoxic area detection reagent;
FIG. 6 is a graph illustrating an expression level of an E-cadherin gene;
FIG. 7 is a graph illustrating an expression level of an N-cadherin gene;
FIG. 8 is a graph illustrating an expression level of a Vimentin gene; and
FIG. 9 is a graph illustrating an expression level of a ZEB1 gene.

### Best Mode for Carrying Out the Invention

A screening method of the present invention includes (a) a process of culturing cells over a cell culture substrate that can form a spheroid, (b) a process of causing such cells to contact a test substance, (c) a process of evaluating an effect of the test substance acting on a maintenance of the epithelial properties of the cells with a change in the morphologies of the spheroid being as an indicator.

A spheroid according to the present invention means an aggregation of cells which have the cells three-dimensionally gathered and aggregated.

A maintenance of the epithelial properties of cells according to the present invention means a condition in which an epithelial-mesenchymal transition is suppressed or inhibited, or a condition in which a mesenchymal-epithelial transition is promoted or induced. Hence, the screening method of the present invention can be roughly divided into a screening method for an epithelial-mesenchymal transition inhibitor, and a screening method for a mesenchymal-epithelial transition inducer.

A screening method for an epithelial-mesenchymal transition inhibitor can be carried out through, for example, a process of culturing cells on a cell culture substrate that can form a spheroid, a process of causing such cells to contact an epithelial-mesenchymal transition inducer, a process of causing the cultured cells to contact a test substance, and a process of evaluating an effect of the test substance acting on an epithelial-mesenchymal transition of the cells with a change in the morphology of the spheroid being as an indicator. An epithelial-mesenchymal transition inducer available is, for example, TGF-β, TNF-α, EGF, or IL-4. The process of causing the cultured cells to contact the epithelial-mesenchymal transition inducer may be executed in any instant before the process of causing the cultured cells to contact the test substance is executed, and for example, may be executed at the time of cell seeding, may be executed when several days have elapsed after the cell seeding, or may be executed simultaneously with the process of causing the cultured cells to contact the epithelial-mesenchymal transition inducer.

A screening method for a mesenchymal-epithelial transition inducer can be carried out through, for example, a process of culturing cells on a cell culture substrate that can form a spheroid, a process of causing the cultured cells to contact a test substance, and a process of evaluating an effect of the test substance acting on a mesenchymal-epithelial transition of the cells with a change in the morphology of the spheroid being as an indicator. According to such a method, it is preferable to carry out such a method while using a cell having a low E-cadherin expression level, such as MIAPaCa-2, PANC-1, or A1165 which is a cell strain derived from a human pancreatic cancer. The test substance may be contacted with the cultured cells after the process of causing the cultured cells to contact the epithelial-mesenchymal transition inducer to induce the cultured cells to be mesenchymal cells. When, in particular, cells of an epithelial cancer (e.g., A549) or a linear cancer (e.g., Capan-2), etc., are used, it is preferable to execute a process by the epithelial-mesenchymal inducer at the time of cell seeding.

An evaluation using a change in a morphology of a spheroid as an indicator according to the present invention can be carried out by comparing differences in external shapes and/or internal structures of spheroids between a negative control group and a test sample group.

The difference in an appearance can have an indicator that is a change in a shape (a circularity), size, and number, etc., of the spheroid. When, for example, the size of the spheroid in the test sample group becomes large in comparison with that of the spheroid in the negative control group, when the number of the spheroids having a size equal to or larger than a predetermined size increases, or when the circularity of the spheroid becomes further closer to 1, it can be evaluated that the test substance is a substance having an effect of maintaining the epithelial properties of cells. Moreover, instead of (or together with) the indicator that is a change in the number of spheroids having a size equal to or larger than the predetermined size, a change in the number of cells that do not form a spheroid can be used as an indicator. This is because such an event can be regarded as a form of a change in the morphology of the spheroid.

Conversely, the difference in an internal structure can have an indicator that is a change in a hypoxic area or an oxygen concentration in the spheroid. When, for example, the hypoxic area in the spheroid in the test sample group is large in comparison with that of the spheroid in the negative control group, a spheroid having a tight cell-to-cell adhesion between cells is formed, and thus it can be evaluated that the test substance have an effect of maintaining the epithelial properties of cells.

Since the screening method of the present invention can capture the difference in the external shape and the difference in the internal structure thereof at the same time, data having a high credibility can be obtained. Moreover, since the method of the present invention can perform evaluation based on a visual parameter, it becomes possible to easily trace a change over time.

A measurement of the difference in the external shape of the spheroid morphology can be carried out through an observation and measurement using a biological microscope like a phase-contrast microscope, and can be also carried out through a plate reader, a device that can measure a three-dimensional shape, and an analysis algorithm for image data, etc.

Conversely, a measurement of the difference in the internal structure is not limited to any particular technique as long as a hypoxic area is detectable, and for example, can be carried out using a compound emitting phosphorescence. When the phosphorescent compound is used, a hypoxic condition can be made visible through a quenching phenomenon of phosphorescence by oxygen, and the hypoxic area and an oxygen concentration can be grasped. Such a compound is not limited to any particular one as long as it emits phosphorescence and causes the quenching phenomenon by oxygen, and it is preferable that such a compound should emit phosphorescence of a long wavelength having a high cell permeability, have a long phosphorescence life and have a high phosphorescence quantum yield. An example compound is an iridium complex having Ir(III) as a major metal and having molecules of aromatic series as ligands, and more specifically, is Bis(2-benzo[b]thiophen-2-yl-pyridine)(acetylacetonate)iridium(III). Since a phosphorescence signal can be quantified, an inhibitory efficiency in an epithelial-mesenchymal transition of the test substance or an induction efficiency in a mesenchymal-epithelial transition of the test substance can be easily obtained.

A cell culture substrate that can form a spheroid according to the present invention is not limited to any particular one as long as an adherence property with a cell is suppressed in comparison with a cell culture substrate used in a normal monolayer culturing, and for example, a cell culture substrate having a hydrophilic property or a hydrophobic property reformed can be used.

A material of a cell culture substrate is not limited to any particular one as long as it is nontoxic to cells, and for example, "polystyrene", "polyethylene", "polypropylene", "polyimide", a "biodegradable polymer, such as polyactic acid, polyactic-acid-polyglycolic-acid copolymer, or polycaprolactone", a "poly olefin resin like polymethylpentene", a "cyclic-olefin-based thermoplastic resin, such as a cyclic olefin copolymer (COC) or a cyclic olefin polymer (COP)", an "acrylic resin", "other resins, such as a photo-curable resin or a thermoset resin", a "metal like aluminum oxide", a "glass", a "quartz glass", or a"silicone" can be used. Moreover, one having a coating layer, such as a "resin", a "photoresist", or a "metal like aluminum oxide", on the surface of a basal plate main body formed of a silicone or glass, etc., can be used.

A treatment for controlling the adherence of cells, such as ultraviolet irradiation, gamma-ray irradiation, plasma irradiation, or coating with various kinds of substances, may be performed on the surface of a cell culture substrate as long as it may function as a cell adhesion surface.

The screening method of the present invention may be carried out using a cell culture substrate having a predetermined concavo-convex structure that functions as a cell adhesion surface.

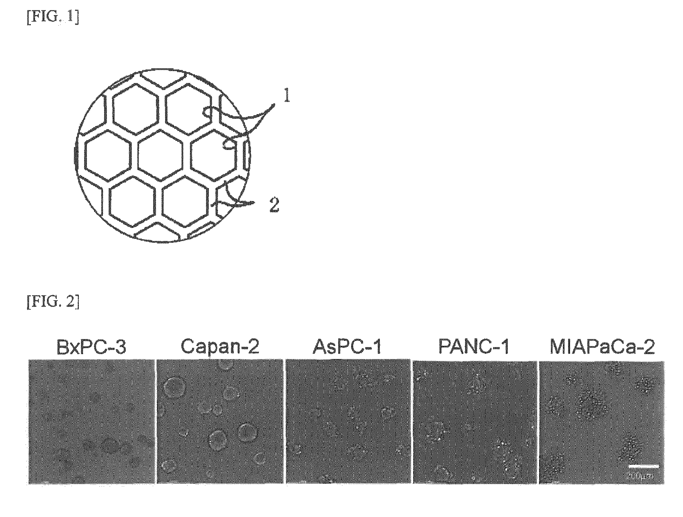

A concavo-convex structure can be formed in various shapes, such as a linear shape (line and space), a pillar shape, or a hole shape in accordance with the properties of cells to be cultured, but a structure having a plurality of unit structures 1 each formed in a predetermined planar shape and arranged regularly is preferable. As shown in FIG. 1, for example, the plurality of unit structures 1 each formed in a polygonal planar shape can be disposed sequentially. In this case, from the standpoint of a cell growth on an isotropically uniform structure, a regular polygon, such as a regular triangle, a square, or a regular hexagon, or a circle is preferable. Moreover, it is possible to combine a pillar or hole shaped concavo-convex structure with a concavo-convex structure formed of the unit structure 1 in a planar shape. From the standpoint of causing cultured cells to be in a condition similar to a condition in a biological object, it is preferable that a line 2 should have a width, such as equal to or smaller than 3 µm, equal to or smaller than 2 µm, equal to or smaller than 1 µm, equal to or smaller than 700 nm, equal to or smaller than 500 nm, or equal to or smaller than 250 nm, and the smaller width is preferable. This is because that it is thought that the smaller the width of the line 2 is, the more cells adhered to a concavo-convex structure plane can grow pseudopods while forming a spheroid.

The unit structure 1 is formed so as to have a depth in various sizes, such as equal to or larger than 1 nm, equal to or larger than 10 nm, equal to or larger than 100 nm, equal to or larger than 200 nm, equal to or larger than 500 nm, equal to or larger than 1 µm, equal to or larger than 10 µm, or equal to or larger than 100 µm, in accordance with a properties of cells to be cultured. Moreover, various aspect ratios of such a concavity and convexity can be adopted, such as equal to or larger than 0.2, equal to or larger than 0.5, equal to or larger than 1, equal to or larger than 2.

It is preferable that the unit structure 1 should have the minimum internal diameter (preferably, the maximum internal diameter) of equal to or smaller than 3 µm, and like equal to or smaller than 2 µm, equal to or smaller than 1 µm, equal to or smaller than 700 nm, equal to or smaller than 500 nm, or equal to or smaller than 250 nm, the smaller diameter is preferable. In this example, an internal diameter means a distance between two parallel lines contacting the external sides of the unit structure 1. Accordingly, the minimum internal diameter means the shortest distance among distances between the two parallel lines contacting the external sides of the unit structure 1, and the maximum internal diameter means the longest distance among the distances between the two parallel lines contacting the external sides of the unit structure 1. When, for example, the unit structure 1 is a regular hexagon, a distance between parallel sides facing with each other is the minimum internal diameter, and a distance between vertices facing with each other is the maximum internal diameter. Moreover, when the unit structure 1 is a rectangle, the length of a short side is the minimum diameter, and the length of a diagonal line is the maximum diameter.

A formation technique of the concavo-convex structure is not limited to any particular one, but for example, nanoimprinting, solution-casting, etching, blasting, or corona discharging can be applied. In this case, from the standpoint of controlling a shape, etc., further highly precisely, a technique through the nanoimprinting is preferable.

Hereinafter, the present invention will be explained in detail with reference to examples. The present invention is, however, not limited to the following description.

### First Example

### [Correlation between E-cadherin Expression Level and Spheroid Morphology]

### (1) Cell Seeding and Observation

Cells were seeded in a cell culture vessel NanoCulture (registered trademark) dish (made by SCIVAX corporation, 35 mm dish, material of concavo-convex structure surface = polymethylpentene (TPX made by MITSUI Chemical Co., Ltd.), planar shape of concavo-convex structure = square, width between unit structures (line width) = 700 nm, minimum internal diameter of unit structure = 3 µm, and depth = 1 µm) at 2.5 × 10⁵ cells, and were observed through an optical microscope at the third day of culturing. The cells used were a human pancreatic cancer cell strain, BxPC-3, Capan-1, Capan-2, AsPC-1, PANC-1, and MIAPaCa-2. The culture media used were a 10-%-FBS-containing RPMI1640 culture medium for BxPC-3, AsPC-1, and PANC-1, a 10-%-FBS-containing MEM culture medium for Capan-1, a 10-%-FBS-containing McCoy's 5A culture medium for Capan-2, and a 10-%-FBS-containing DMEM culture medium for MIAPaCa-2.

### (2) Western Blotting

The same cells as those of (1) were also seeded in the same cell culture vessel NanoCulture (registered trademark) dish (made by SCIVAX corporation, 35 mm dish) as that of (1), and cultured for five days, and those cells were collected by a pipette. The collected cells were rinsed by PBS, dissolved in a dissolving buffer, and cell dissolved solutions were taken as samples. Next, a sample of each cell strain (corresponding to 20 µg protein) was separated through SDS poly-acrylamide gel electrophoresis (SDS-PAGE), and protein was transferred to a PVDF film. A detection of protein was carried out using mouse anti-E-cadherin monoclonal antibody (BD Biosciences: Cat #610182) as a primary antibody, an anti-mouse antibody having undergone HRP labeling (Santa Cruz: sc-2005) as a secondary antibody, and an ECL Plus Western Blotting Detection System (made by GE healthcare corporation) as a detection reagent.

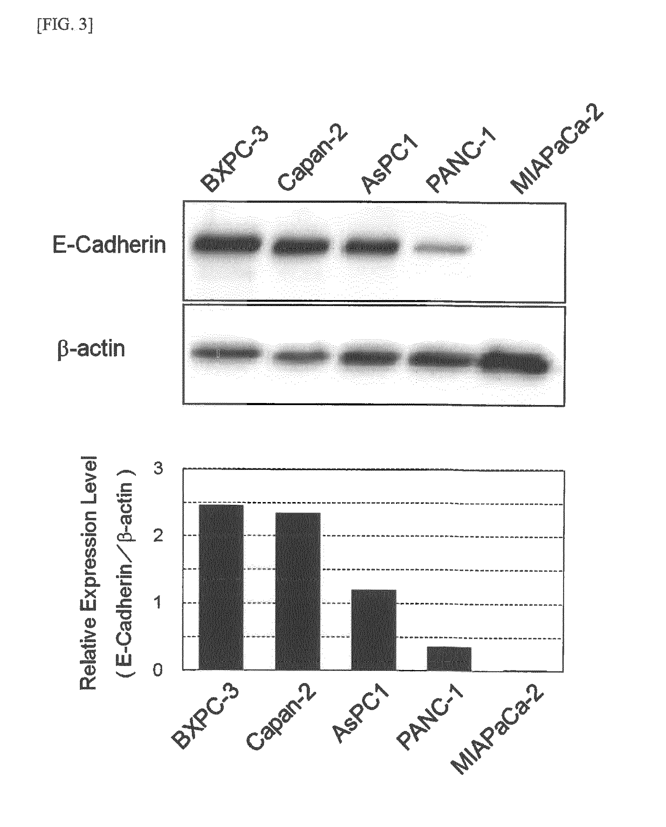

The results of (1) and (2) are shown in FIGS. 2 and 3. According to BxPC-3 and Capan-2 having a high expression level of E-cadherin, the circularity of spheroid was close to 1, and it becomes clear that a spheroid having a tight cell-to-cell adhesion was formed. In contrast, according to PANC-1 and MIAPaCa-2 having a low expression level of E-cadherin, the circularity was apart from 1 more than the spheroid of the above-explained cell strains, and it becomes clear that a spheroid having loose cell adhesion was formed.

### Second Example

### [Effect of Epithelial-mesenchymal Transition Inhibitor Acting on Morphology of Spheroid]

### (1) Pre-incubation

5-%-FBS-containing DMEM culture medium and 1.0%-Matrigel (registered trademark) containing DMEM culture medium were added in respective wells of a cell culture vessels NanoCulture (registered trademark) plate (made by SCIVAX corporation, 384-well plate, material of concavity-convexity structure surface = polymethylpentene (TPX made by MITSUI Chemical Co., Ltd.), planar shape of concavo-convex structure = square, width between unit structures (line width) = 700 nm, minimum internal diameter of unit structure = 3 µm, and depth = 1 µm) by 25 µl, and were subjected to centrifugal force for five minutes at 1000 × g.

### (2) Cell Seeding

A cell suspension having human-lung-cancer-cells derived strain A549 adjusted to 13.5 × 10⁴ cells/ml by a 5-%-FBS-containing DMEM culture medium was added to each well of the above-explained plate by 25 µl (the number of cells was 3.4 × 10³ cells/well, and the final concentration of the culture medium additive was 5% FBS and 0.5% Matrigel).

### (3) Addition of Epithelial-mesenchymal Transition Inducer and Inhibitor

At the third day of the culturing, TGF-β2 (made by R&D systems corporation, 302-B2-002) and TNF-α (made by R&D systems corporation, 210-TA-010) which were epithelial-mesenchymal transition inducers were added to each well in such a way that a final concentration became 5 ng/ml and 10 ng/ml, respectively While at the same time, SB431542 (made by Miltenyi Biotec corporation, 130-095-561) that was an inhibitor of TGF-β2 was added in such a way that a final concentration became 10 µM. Dimethyl-sulfoxide was added to all wells where no SB431542 was added in such a way that a final concentration became 0.1 %.

### (4) Addition of Hypoxic Area Detection Reagent

At the fifth day of the culturing, a hypoxic area detection reagent LOX-1 (made by SCIVAX corporation) was added to each well in such a way that a final concentration became 2 µM. LOX-1 is a reagent that contains iridium complex which emits red phosphorescence.

### (5) Observation

An observation through a fluorescent microscope was carried out at the sixth day of the culturing.

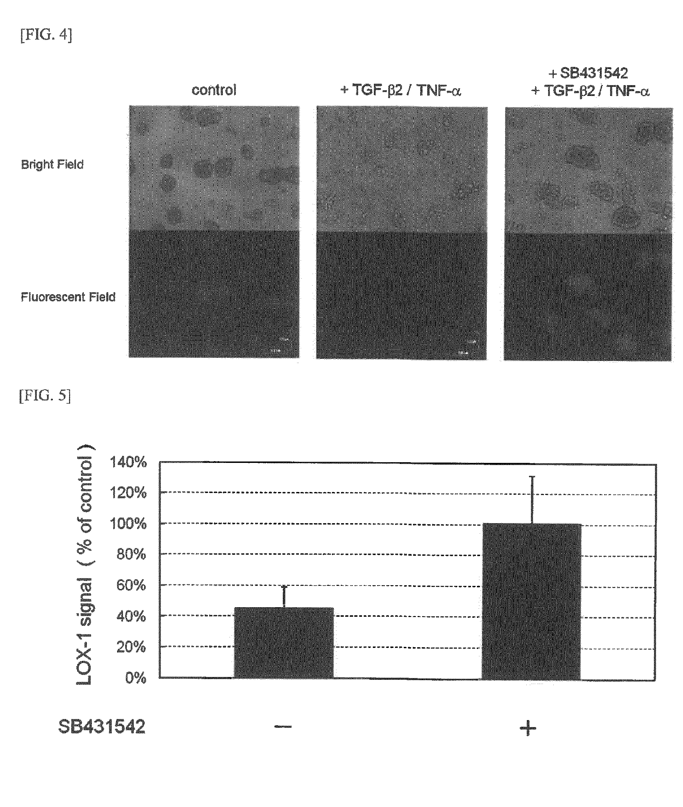

A result obtained through an observation and image-pickup by the fluorescent microscope is shown in FIG. 4. First of all, when bright field images of respective wells are compared with each other, in the case of the well (a control) where no epithelial-mesenchymal transition inducer was added, compact spheroids having a circularity close to 1 were formed. In contrast, in the case of the well where the epithelial-mesenchymal transition inducers were added, it becomes clear that the majority of spheroids were collapsed, and the contours of the remaining spheroids have a large concavity and convexity. In the case of the well where both epithelial-mesenchymal transition inducer and inhibitor were added, it becomes clear that in comparison with the well where no inhibitor was added, the collapse of spheroids were suppressed and the contours of the spheroids had a small concavity and convexity. Conversely, when fluorescent field images of respective wells are compared with each other, it becomes clear that in the case of the well where the epithelial-mesenchymal transition inducer was added, in comparison with the control, the red intensity was small and the red area was small, and in the case of the well where the inhibitor was further added, the red intensity was high and the red area was large. That is, it becomes clear that a hypoxic area was large. A result of quantifying the red signal at this time is shown in FIG. 5. It becomes clear that in the case of the well where the epithelial-mesenchymal transition inhibitor was added, the red signal was about twice as much as that of the well where no inhibitor was added.

### Third Example

### [Verification of Change in Gene Expression at the time of adding Epithelial-mesenchymal Transition Inducer and Inhibitor]

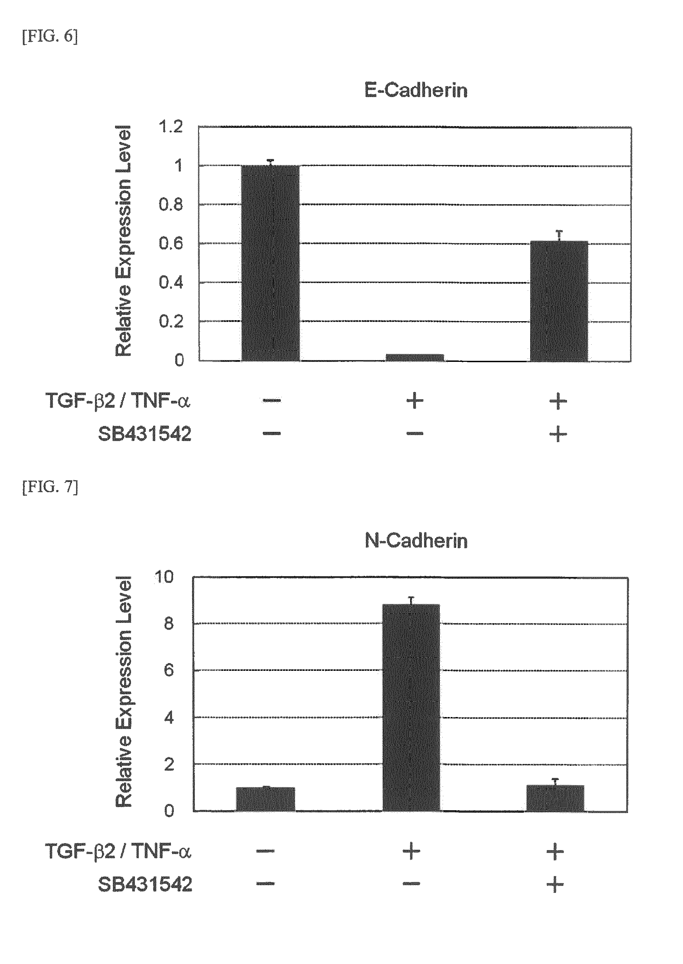

In order to verify whether or not a change in the morphology of the spheroid confirmed in the second example reflects an epithelial-mesenchymal transition and an epithelial-mesenchymal transition inhibition, a result of measuring an expression level of a gene that is known as an epithelial or mesenchymal marker through qRT-PCR is shown in FIGS. 6 to 9. The processes from (1) pre-incubation to (3) addition of the epithelial-mesenchymal transition inducer and inhibitor were carried out likewise the second example. Next, at the sixth day of the culturing, all RNAs were extracted from each sample using RNeasy Plus Mini Kit (made by QIAGEN corporation), and such RNAs having undergone reverse transcription using PrimeScript RT reagent Kit (made by TAKARABIO Inc.) were used for qRT-PCR. SYBER Premix Ex Taqll (made by TAKARABIO Inc.) was used for PCR. Moreover, Thermal Cycler Dice (made by TAKARABIO Inc.) was used for a reaction and a detection. The primers used for PCR are shown in the following table 1. Note that data is corrected by the expression level of internal standard gene (TBP), and is shown as a relative value when the expression level of the control is taken as 1.

**[Table 1]**

| Gene Name | Forward Primer | Reverse Primer |
|---|---|---|
| E-Cadherin | Sequence Number 1 | Sequence Number 2 |
| N-Cadherin | Sequence Number 3 | Sequence Number 4 |
| Vimentin | Sequence Number 5 | Sequence Number 6 |
| ZEB1 | Sequence Number 7 | Sequence Number 8 |

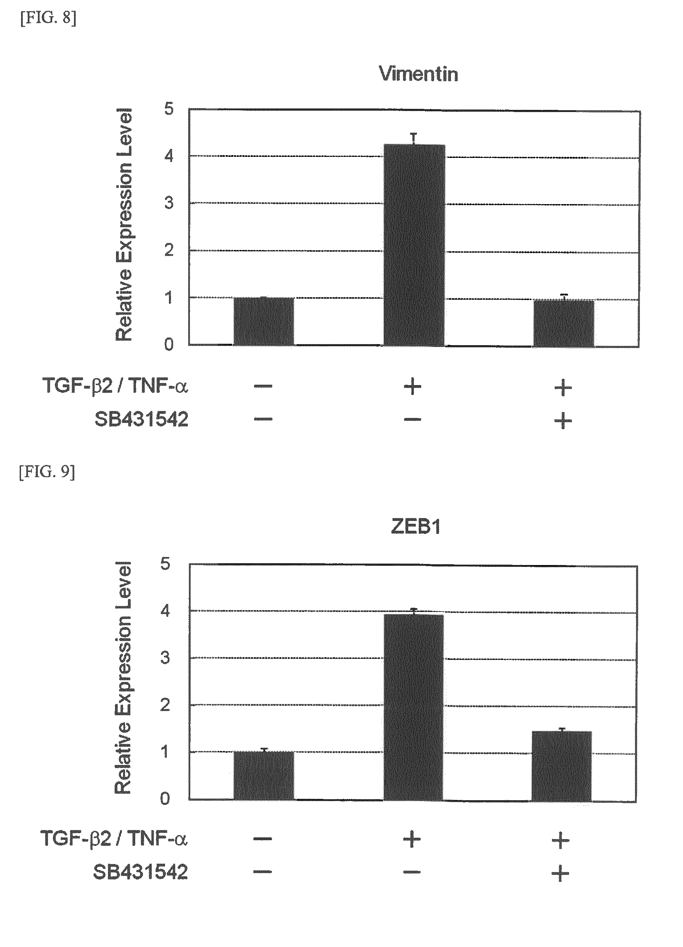

The expression level of E-cadherin known as an epithelial marker was reduced to be equal to or lower than 10 % of the control upon addition of the epithelial-mesenchymal transition inducer, and was recovered to substantially 60 % of the control upon addition of the inhibitor (FIG. 6). Conversely, the expression level of N-cadherin known as a mesenchymal marker was increased to about nine times as much as that of the control upon addition of the epithelial-mesenchymal transition inducer, and decreased to substantially same level as that of the control upon addition of the inhibitor (see FIG. 7). Likewise, the expression of Vimentin that is a mesenchymal marker and that of ZEB1 that is a transcription factor which suppresses an expression of E-cadherin indicated the similar expression level change to that of N-cadherin (FIGS. 8 and 9). It is taught and suggested from those results that a change in the morphology of the spheroids confirmed in the second example reflected a change in the properties of the cells occurring at the time of an epithelial-mesenchymal transition or an epithelial-mesenchymal inhibition.

### Industrial Applicability

The present invention is applicable to a screening of medical agents, such as an anticancer agent and a fibrosis therapeutic medicine.

### Description of Reference Numerals

- 1: Unit structure
- 2: Line

## Claims

1. A screening method for a substance acting on a maintenance of epithelial properties of cells, comprising:
(a) a step for culturing cells on a cell culture substrate that can form a spheroid;
(b) a step for causing the cells to contact a test substance; and
(c) a step for evaluating an effect of the test substance acting on a maintenance of the epithelial properties of the cells with a change in a morphology of the spheroid being as an indicator.

2. The screening method according to claim 1, wherein the step (c) carries out an evaluation through a measurement of a number of spheroids in a predetermined size.

3. The screening method according to claim 1 or 2, wherein the step (c) carries out an evaluation using a reagent that can detect a hypoxic area in a spheroid.

4. The screening method according to any one of claims 1 to 3, wherein the step (a) is carried out on a cell culture substrate comprising a predetermined concavo-convex structure that functions as a cell adhesion surface.

5. The screening method according to claim 4, wherein the concavo-convex structure comprises a plurality of unit structures disposed with regularity, the unit structure being formed in a predetermined planar shape.

6. The screening method according to claim 4, wherein the concavo-convex structure comprises a plurality of unit structures disposed with regularity, a width between the unit structures being equal to or smaller than 3 µm, the unit structure being formed in a polygonal shape in a plane direction, and a minimum internal diameter of the unit structure being equal to or smaller than 3 µm.


SEQUENCE LISTING

<110>  SCIVAX CORPORATION

<120>  Screening method for substances maintaining epithelial integrity

<130>  SP006600PCT

<160>  8

<170>  PatentIn version 3.5

<210>  1
<211>  20
<212>  DNA
<213>  Artificial Sequence

<220>
<223>  PCR Forward primer for E-Cadherin

<400>  1
gaacgcattg ccacatacac                                                    20


<210>  2
<211>  20
<212>  DNA
<213>  Artificial Sequence

<220>
<223>  PCR Reverse primer for E-Cadherin

<400>  2
attcgggctt gttgtcattc                                                    20


<210>  3
<211>  20
<212>  DNA
<213>  Artificial Sequence

<220>
<223>  PCR Forward primer for N-Cadherin

<400>  3
aggtttgcca gtgtgactcc                                                    20


<210>  4
<211>  20
<212>  DNA
<213>  Artificial Sequence

<220>
<223>  PCR Reverse primer for N-Cadherin

<400>  4
ccacaaacat cagcacaagg                                                    20


<210>  5
<211>  20
<212>  DNA

<213>  Artificial Sequence

<220>
<223>  PCR Forward primer for Vimentin

<400>  5
agtaccggag acaggtgcag                                                    20


<210>  6
<211>  20
<212>  DNA
<213>  Artificial Sequence

<220>
<223>  PCR Reverse primer for Vimentin

<400>  6
gcttcaacgg caaagttctc                                                    20


<210>  7
<211>  20
<212>  DNA
<213>  Artificial Sequence

<220>
<223>  PCR Forward primer for ZEB1

<400>  7
ggaaagcgct tctcacactc                                                    20


<210>  8
<211>  20
<212>  DNA
<213>  Artificial Sequence

<220>
<223>  PCR Reverse primer for ZEB1

<400>  8
gtcacgttct tccgcttctc                                                    20